# EUROPEAN PATENT SPECIFICATION

(11) **EP 3 892 208 B1**
(45) Date of publication and mention of the grant of the patent: **22.01.2025**
(21) Application number: 21167096.3
(22) Date of filing: 07.04.2021
(51) Int. Cl.: A61B 17/072, A61B 17/32

(54) **SURGICAL STAPLING DEVICE WITH ADJUSTABLE DISSECTING TIP**
CHIRURGISCHE KLAMMERVORRICHTUNG MIT VERSTELLBARER PRÄPARIERSPITZE
DISPOSITIF D'AGRAFAGE CHIRURGICAL À POINTE DE DISSECTION RÉGLABLE

(30) Priority: 07.04.2020 US 202016842016
(43) Date of publication of application: 13.10.2021
(73) Proprietor: Covidien LP, Mansfield, MA 02048 (US)
(72) Inventor: WALCOTT, Zalika, Hamden, Connecticut 06514 (US); ABRAMEK, Pawel, Berlin, Connecticut 06037 (US); CAPPOLA, Kenneth M., Monroe, Connecticut 06468 (US)
(74) Representative: Maschio & Soames IP Ltd

(56) References cited:
- EP-A1- 3 138 509
- EP-A1- 3 689 265
- US-A1- 2018 235 610
- US-A1- 2018 325 514
- US-A1- 2020 054 323

## Description

### FIELD

The disclosure is directed to surgical stapling devices and, more particularly, to endoscopic surgical stapling devices with dissecting tips.

### BACKGROUND

Surgical stapling devices for performing surgical procedures endoscopically are well known. Such devices are available in a variety of different configurations, e.g., linear, curved, circular, etc., and are suitable for use in a variety of different procedures. Linear surgical stapling devices include a tool assembly having an anvil and a staple cartridge that are pivotably coupled to each other at their proximal ends between open and clamped positions. In order to better navigate the tool assembly to a surgical site endoscopically, the tool assembly may include a dissector tip that extends from a distal end of the tool assembly. Typically, the dissector tip is supported on the tool assembly and is configured to separate target tissue from body tissue to facilitate placement of the tool assembly about the target tissue. During some surgical procedures, it is desirable to have an angled dissector tip whereas in other surgical procedures it is desirable to have a linear dissector tip.

A continuing need exists in the art for a surgical stapling device that is better suited to access to a variety of surgical sites.

Document EP 3,689,265 A1, a document falling under Article 54(3) EPC, discloses a surgical instrument end effector including a tip member movably disposed at a distal end of an anvil and is configured to toggle relative to the anvil between a first discrete position and a second discrete position. Documents US 2018,325,514, US 2018,235,610, EP 3,138,509 and US 2020,054,323 disclose other prior art surgical stapling instruments with a movable tip.

### SUMMARY

One aspect of this disclosure is directed to a claimed surgical stapling device according to claim 1. Further developments of the claimed invention can be gathered from the dependent claims.

Other features of the disclosure will be appreciated from the following description.

### BRIEF DESCRIPTION OF THE DRAWINGS

Various aspects of the disclosed surgical stapling device are described herein below with reference to the drawings, wherein:
FIG. 1 is a side perspective view of the disclosed surgical stapling device including a tool assembly in an open position having a dissecting tip according to exemplary aspects of the disclosure;
FIG. 2 is an enlarged view of the indicated area of detail shown in FIG. 1 illustrating the tool assembly in the open position;
FIG. 3 is a side perspective view of the tool assembly of the surgical stapling device shown in FIG. 1 with the tool assembly in the clamped position;
FIG. 4 is a side perspective view of an anvil assembly of the tool assembly shown in FIG. 3 with a dissecting tip separated from the anvil assembly;
FIG. 5 is a perspective view from the distal end of the anvil assembly shown in FIG. 4;
FIG. 6 is cross-sectional view taken along section line 6-6 of FIG. 5;
FIG. 7 is a side perspective view of the dissector tip of the surgical stapling device shown in FIG. 4;
FIG. 8 is a cross-sectional view taken along section line 8-8 of FIG. 3;
FIG. 9 is an enlarged view of the indicated area of detail shown in FIG. 8 illustrating the dissecting tip in an angled position;
FIG. 10 is a side perspective view of the distal end of the tool assembly shown in FIG. 3 in the clamped position with the dissecting tip in an aligned position;
FIG. 11 is cross-sectional view taken along section line 11-11 of FIG. 10;
FIG. 12 is a side perspective view of another version of the anvil assembly of the tool assembly of the surgical stapling device shown in FIG. 1 with the dissecting tip separated from the anvil assembly;
FIG. 13 is cross-sectional view taken through the dissecting tip and the anvil assembly shown in FIG. 12 with the dissecting tip in an angled position; and
FIG. 14 is cross-sectional view taken through the dissecting tip and the anvil assembly shown in FIG. 12 with the dissecting tip in an aligned position.

### DETAILED DESCRIPTION

The disclosed surgical stapling device will now be described in detail with reference to the drawings in which like reference numerals designate identical or corresponding elements in each of the several views. However, it is to be understood that the aspects of the disclosure described herein are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally to refer to endoscopic, laparoscopic, arthroscopic, and/or any other procedure conducted through a small diameter incision or cannula. Further, the term "clinician" is used generally to refer to medical personnel including doctors, nurses, and support personnel. As used herein, the terms "parallel" and "aligned" are understood to include relative configurations that are substantially parallel, and substantially aligned, i.e., up to about + or - 10 degrees from true parallel or true alignment.

The disclosed surgical stapling device includes a tool assembly that supports a dissector tip that is adjustable to allow a clinician to position the dissector tip in a configuration that facilitates easy access to a surgical site. Some aspects of the disclosure are directed to a dissector tip that is pivotable between a first orientation in which the axis defined by the dissector tip is parallel to a longitudinal axis of the tool assembly and a second position in which the axis defined by the dissector tip defines an acute angle with the longitudinal axis of the tool assembly.

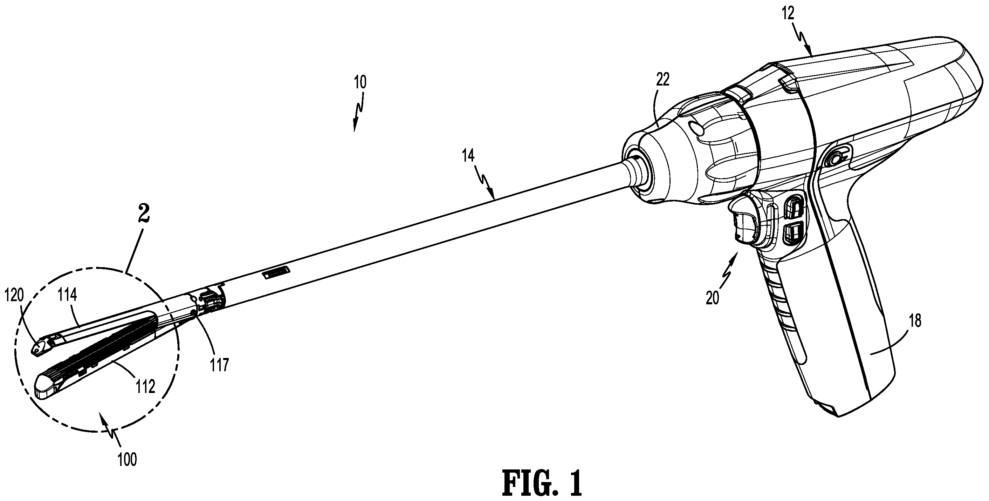

FIG. 1 illustrates the disclosed surgical stapling device which is shown generally as stapling device 10 and includes a handle assembly 12, an elongate body 14, and a tool assembly 100. The handle assembly 12 includes a hand grip 18 and a plurality of actuator buttons 20 and supports a rotation knob 22. The rotation knob 22 supports the elongate body 14 and is rotatable in relation to the handle assembly 14 to facilitate rotation of the elongate body 14 and the tool assembly 100 in relation to the handle assembly 12. The actuator buttons 20 control operation of the various functions of the stapling device 10 including approximation, firing and cutting. The tool assembly 100 can be secured directly to a distal portion of the elongate body 14. Alternately, the tool assembly 100 can form part of a reload assembly that is releasably coupled to the elongate body 14 and can be replaced to facilitate reuse of the surgical stapling device. Although the stapling device 10 is illustrated as an electrically powered stapling device, it is envisioned that the disclosed tool assembly 100 is suitable for use with a manually powered surgical stapling device. U.S. Patent No. 9,055,943 (the '943 Patent) discloses a stapling device including a powered handle assembly and U.S. Patent No. 6,241,139 (the '139 Patent) discloses a manually actuated handle assembly.

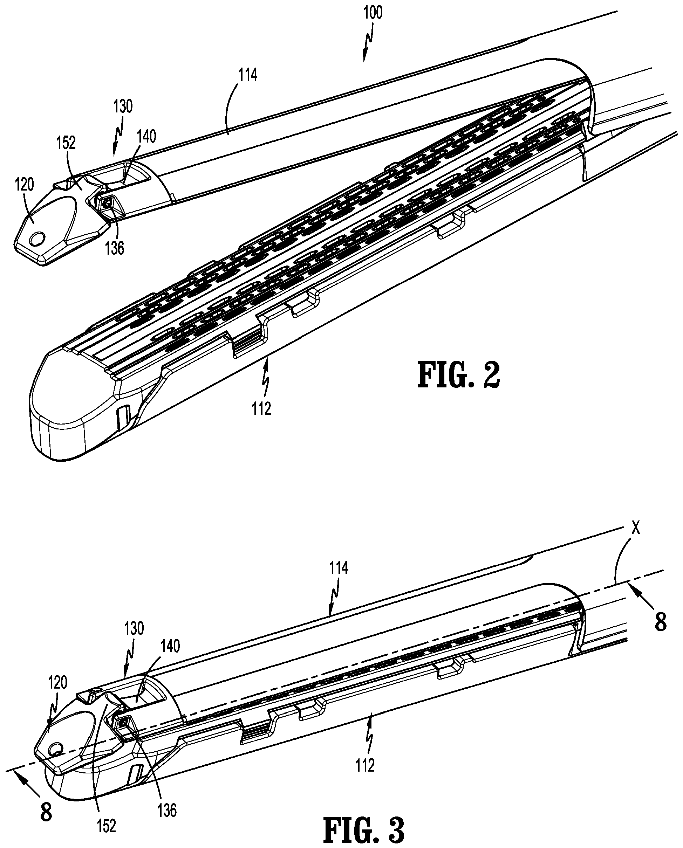

FIGS. 1-3 illustrate the tool assembly 100 which defines a longitudinal axis "X" (FIG. 3) and includes a cartridge assembly 112, an anvil assembly 114, and a dissector tip 120 that is supported on the anvil assembly 114. The cartridge assembly 112 is secured to the anvil assembly 114 for pivotal movement between an open position and a clamped position. For a more detailed description of the cartridge assembly 112 of the tool assembly 100, see, e.g., the '943 and ' 139 Patents. Although the tool assembly is illustrated as a stapling device, it is envisioned that the tool assembly could include a variety of different devices including graspers, electrical tissue sealing/cutting devices, etc.

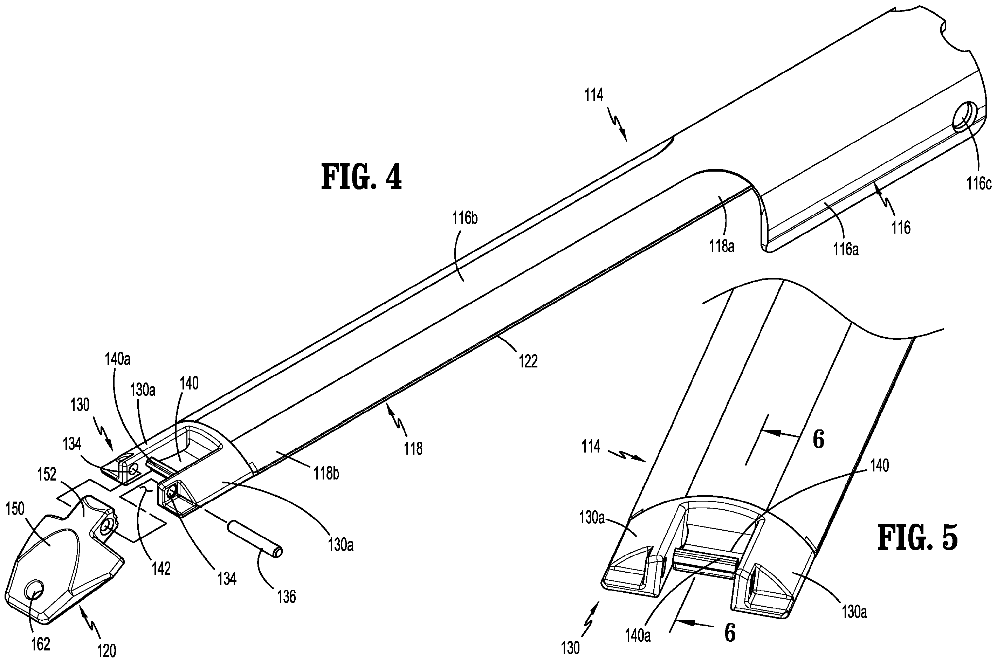

FIGS. 4-7 illustrate the anvil assembly 114 and dissector tip 120 of the tool assembly 100. The anvil assembly 114 includes a base portion 116 and an anvil portion 118. The base portion 116 includes a proximal mounting portion 116a and a longitudinal body portion 116b that extends distally from the proximal mounting portion 116a. The proximal mounting portion 116a includes spaced through bores 116c that receive pivot members 117 (FIG. 1) that pivotably couple the cartridge assembly 112 to the anvil assembly 114. The anvil portion 118 is secured to the base portion 116 by, e.g., welding, and includes a staple forming surface 122, a proximal portion 118a, and a distal portion 118b. The staple forming surface 122 of the anvil portion 118 defines a plurality of rows of staple deforming pockets (not shown) that are positioned on opposite sides of a knife slot (not shown). The distal portion 118b of the anvil portion 118 includes a clevis 130 that has spaced arms 130a. Each of the spaced arms 130a defines an opening 134 that receives a clevis pin 136. The distal portion 118b of the anvil portion 118 includes a distal face 138 that supports a retaining member. In aspects of the disclosure, the retaining member includes a resilient cantilevered finger 140 that supports a protrusion 140a. The spaced arms 130a define a cavity 142 and the clevis pin 136 extends through the cavity 142 between the spaced arms 130a of the clevis 130.

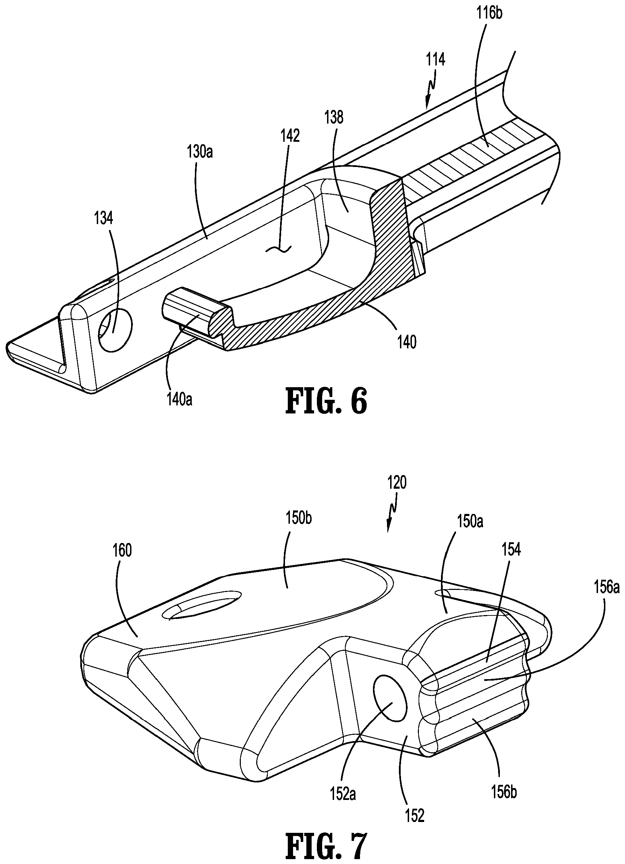

The dissector tip 120 has a body 150 (FIG. 4) that defines a longitudinal axis "Y" and includes a proximal portion 150a and a distal portion 150b. The proximal portion 150a includes a hinge portion 152 that defines a through bore 152a that receives the clevis pin 136 to pivotably secure the dissector tip 120 to the distal end portion 118b of the anvil portion 118 of the anvil assembly 114. The hinge portion 152 of the dissector tip 120 has a proximal face 154 that defines spaced recesses 156a and 156b, each of which is positioned to receive the protrusion 140a on the cantilevered finger 140 of the anvil portion 118 of the anvil assembly 114 (FIG. 6) as described below. The dissector tip 120 has an angled distal face 160 that extends towards the cartridge assembly 112 in the distal direction (FIG. 3) such that the distal end of the dissector tip 120 has a height that decreases in the distal direction. In aspects of the disclosure, the width of the dissector tip 120 decreases in the distal direction (FIG. 4). Alternately, the dissector tip 120 can have a variety of different configurations that allow the tool assembly 100 to access target tissue.

In some aspects of the disclosure, the dissector tip 120 has a through bore 162 (FIG. 4) that extends through the tapered distal face 160. The through bore 162 allows a clinician to lasso or grasp the dissector tip 120 with a suture to allow the clinician to more accurately move and position the tool assembly 100 within a body cavity during a surgical procedure.

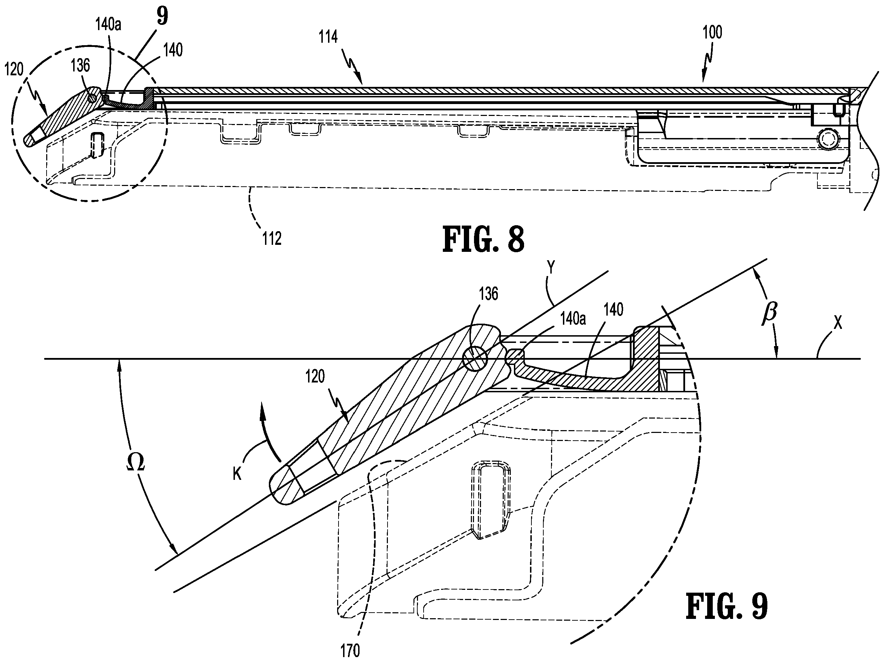

The dissector tip 120 pivots from a first position to a second position about the clevis pin 136. FIGS. 8 and 9 illustrate the dissector tip 120 in the first position. In the first position, the protrusion 140a of the cantilevered finger 140 is received within the recess 156b on the proximal surface 154 of the hinge portion 152 of the dissector tip 120 to retain the dissector tip 120 in the first position. In the first position, the longitudinal axis "Y" of the dissector tip 120 and the longitudinal axis "X" of the tool assembly 100 define an acute angle Ω (FIG. 9). It is envisioned that angle Ω can be substantially the same as an angle β defined by a tissue guide surface 170 of the cartridge assembly 112 and the longitudinal axis "X". Alternately, other angles are envisioned.

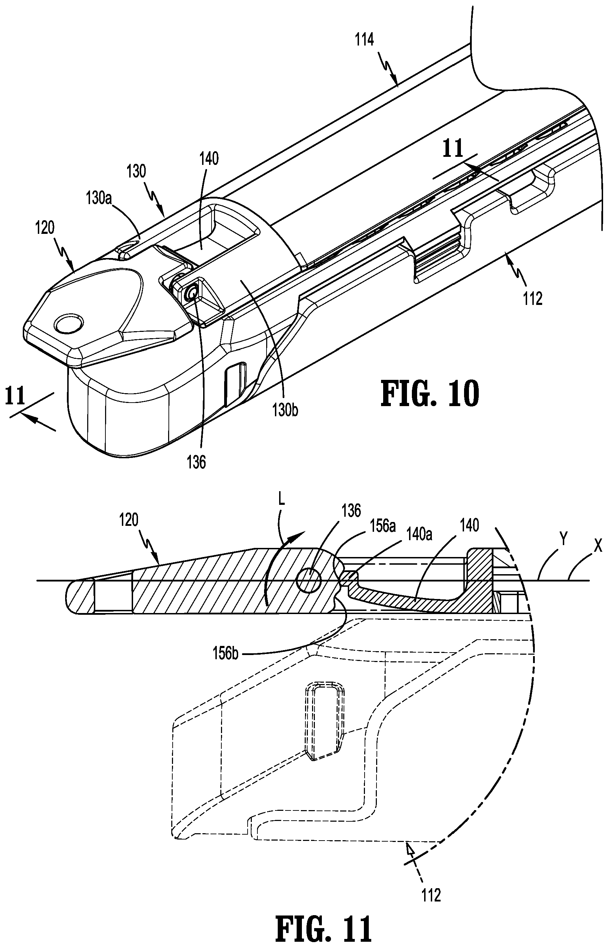

FIGS. 10 and 11 illustrate the dissector tip 120 in the second position. In the second position, the longitudinal axis "Y" of the dissector tip 120 is substantially parallel to or coaxial with the longitudinal axis "X of the tool assembly 100. In order to move the dissector tip 120 between the first and second positions, a clinician can apply a force to the dissector tip 120 to disengage the protrusion 140a from within one of the respective spaced recesses 156a and 156b and pivot the dissector tip 120 about the clevis pin 136 to position the protrusion 140a in the other recess 156a or 156b of the dissector tip 120. For example, when the dissector tip 120 is moved from the first position (FIG. 8) to the second position (FIG. 10), the clinician applies a force on the dissector tip 120 in the direction indicated by arrow "K" in FIG. 9 to pivot the dissector tip 120 about the clevis pin 136 in the direction indicated by arrow "L" in FIG. 11 to remove the protrusion 140a of the cantilevered finger 140 from the recess 156b of the dissector tip 120 and move the protrusion 140a into the recess 156a of the dissector tip 120. The cantilevered finger 140 is flexible to allow the protrusion 140a to move between the recesses 156a-b. Although only two positions are shown in the figures, it is envisioned that the dissector tip 120 may be moved between multiple positions. In that respect, it is envisioned that two or more spaced recesses 156a-b can be provided to receive the protrusion 140a of the cantilevered finger 140 to retain the dissector tip 120 in one of multiple different angular positions in relation to the longitudinal axis "X" of the tool assembly 100.

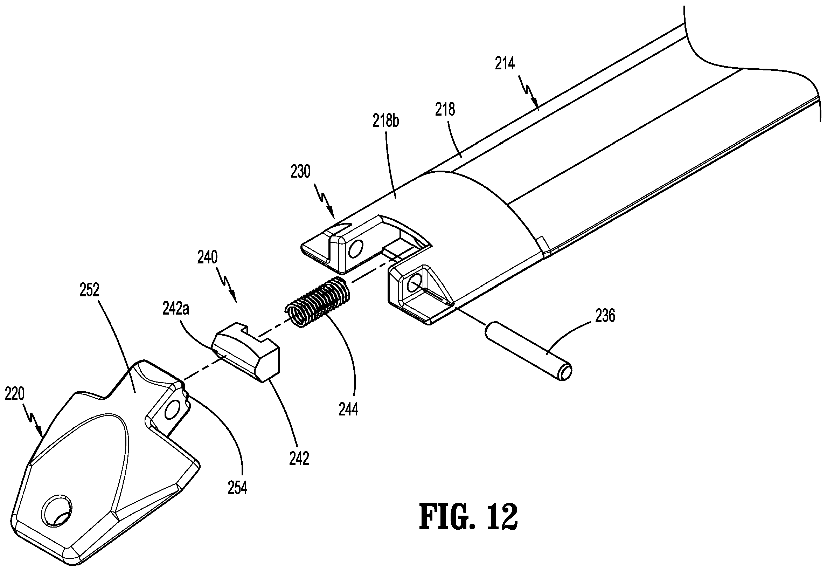

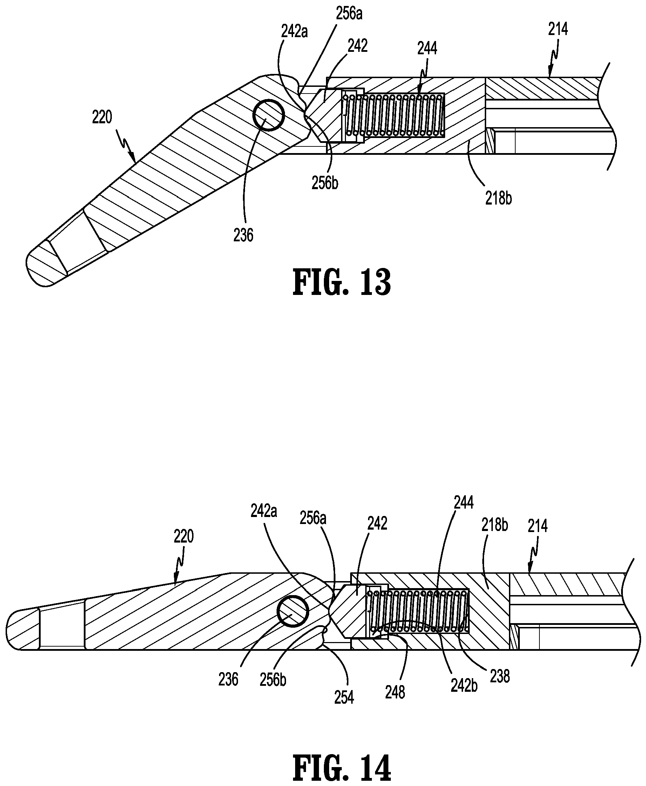

FIGS. 12-14 illustrate an alternative version of the anvil assembly shown generally as anvil assembly 214. The dissector tip 220 is substantially as described above and includes a hinge portion 252 that includes a proximal face 254 that defines spaced recesses 256a and 256b. The hinge portion 252 is supported within a clevis 230 (FIG. 12) of the anvil assembly 214 by a clevis pin 236. In the anvil assembly 214, the retaining member on the distal portion 218b (FIG. 12) of the anvil portion 218 (FIG. 12) includes a plunger mechanism 240 that includes a plunger 242 and a biasing member 244. The plunger 242 includes a protrusion 242a that is received within one of the recesses 256a and 256b on the proximal face 254 of the dissector tip 220 to retain the dissector tip 220 in one of the first position (FIG. 13) and the second position (FIG. 14). The plunger 242 is received within a blind bore 248 formed in a distal face 238 of the anvil portion 218. The biasing member 244 which may be in the form of a coil spring is positioned within the blind bore 248 and a bore 242b formed in a proximal side of the plunger 242 to urge the plunger 242 distally into engagement with the hinge portion 252 of the dissector tip 220.

Although the drawings only show retaining structure including a protrusion and a plurality of recesses, it is also envisioned that a variety of different types of retention structures can be provided to releasably retain the dissector tips 120 and 220 in different angular positions. Further, the retaining structure can be supported on or integrally formed with either or both dissection tip 220 and the anvil assembly 214.

Persons skilled in the art will understand that the devices and methods specifically described herein and illustrated in the accompanying drawings are non-limiting exemplary aspects of the disclosure. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

## Claims

1. A surgical stapling device (10) comprising:
an elongate body (14) having a proximal portion and a distal portion; and
a tool assembly (100) defining a longitudinal axis and including:
a cartridge assembly (112);
an anvil (114, 118) including a proximal portion and a distal portion, the anvil coupled to the cartridge assembly such that the tool assembly is moveable from an open position to a clamped position;
a dissector tip (120) including a body having a proximal portion (150a) and a distal portion (150b), the body having a thickness that decreases towards the distal portion of the body of the dissector tip, the body being movably coupled to the anvil for movement between a first position substantially aligned with the longitudinal axis and a second configuration defining an acute angle with the longitudinal axis; wherein the dissector tip includes a proximal face (154a) that defines spaced recesses (156a, 156b, 256a, 256b),
and
a retaining member supported on the distal portion of the anvil, the retaining member engaged with the spaced recesses of the dissector tip to releasably retain the dissector tip in one of the first or second positions.

2. The surgical stapling device of claim 1, wherein the retaining member includes a resilient cantilevered finger (140) supported on the distal portion of the anvil.

3. The surgical stapling device of claim 2 wherein the cantilevered finger supports a protrusion (140a), the protrusion received within one of the spaced recesses to retain the dissector tip in one of the first or second positions.

4. The surgical stapling device of claim 3, wherein the distal portion of the anvil includes a clevis (130) and the proximal portion of the dissector tip includes a hinge portion (152) that is supported within the clevis by a clevis pin (136).

5. The surgical stapling device of claim 4, wherein the proximal face of the dissector tip is located on the hinge portion of the dissector tip.

6. The surgical stapling device of claim 1, wherein the retaining member includes a plunger (242)
that is releasably engaged with the dissector tip to retain the dissector tip in one of the first or second positions; preferably wherein the retaining member includes a biasing member (244)
that is engaged with the plunger to urge the plunger into engagement with the dissector tip.

7. The surgical stapling device of claim 6, wherein the plunger includes a protrusion (242a), the protrusion received within one of the spaced recesses to retain the dissector tip in one of the first or second positions; preferably wherein the distal portion of the anvil includes a clevis and the proximal portion of the dissector tip includes a hinge portion that is supported within the clevis by a clevis pin.

8. The surgical stapling device of claim 7, wherein the proximal face of the dissector tip is located on the hinge portion of the dissector tip.

9. The surgical stapling device of any preceding claim, further including a handle assembly, the proximal portion of the elongate body coupled to the handle assembly.

## Patentansprüche

1. Chirurgische Klammervorrichtung (10), umfassend:
einen länglichen Körper (14) mit einem proximalen Abschnitt und einem distalen Abschnitt; und
eine Werkzeuganordnung (100), die eine Längsachse definiert und aufweist:
eine Magazinanordnung (112);
einen Amboss (114, 118), der einen proximalen Abschnitt und einen distalen Abschnitt aufweist, wobei der Amboss derart mit der Magazinanordnung gekoppelt ist, dass die Werkzeuganordnung aus einer offenen Position in eine Klemmposition beweglich ist;
eine Dissektorspitze (120), die einen Körper mit einem proximalen Abschnitt (150a) und einem distalen Abschnitt (150b) aufweist, wobei der Körper eine Dicke hat, die in Richtung des distalen Abschnitts des Körpers der Dissektorspitze abnimmt, wobei der Körper zur Bewegung zwischen einer ersten Position, die im Wesentlichen auf die Längsachse ausgerichtet ist, und einer zweiten Auslegung, die einen spitzen Winkel zur Längsachse definiert, beweglich mit dem Amboss gekoppelt ist; wobei die Dissektorspitze eine proximale Fläche (154a) aufweist, die beabstandete Aussparungen (156a, 156b, 256a, 256b) definiert, und
ein Halteelement, das an dem distalen Abschnitt des Ambosses gelagert ist, wobei das Halteelement mit den beabstandeten Aussparungen der Dissektorspitze im Eingriff ist, um die Dissektorspitze in einer von der ersten oder der zweiten Position lösbar zu halten.

2. Chirurgische Klammervorrichtung nach Anspruch 1, wobei das Halteelement einen elastischen freitragenden Finger (140) aufweist, der an dem distalen Abschnitt des Ambosses gelagert ist.

3. Chirurgische Klammervorrichtung nach Anspruch 2, wobei der freitragende Finger einen Vorsprung (140a) stützt, wobei der Vorsprung in einer der beabstandeten Aussparungen aufgenommen ist, um die Dissektorspitze in einer von der ersten oder der zweiten Position zu halten.

4. Chirurgische Klammervorrichtung nach Anspruch 3, wobei der distale Abschnitt des Ambosses einen Gabelkopf (130) aufweist und der proximale Abschnitt der Dissektorspitze einen Gelenkabschnitt (152) aufweist, der durch einen Gabelkopfstift (136) in dem Gabelkopf gehalten wird.

5. Chirurgische Klammervorrichtung nach Anspruch 4, wobei sich die proximale Fläche der Dissektorspitze an dem Gelenkabschnitt der Dissektorspitze befindet.

6. Chirurgische Klammervorrichtung nach Anspruch 1, wobei das Halteelement einen Kolben (242) aufweist, der lösbar mit der Dissektorspitze im Eingriff ist, um die Dissektorspitze in einer von der ersten oder der zweiten Position zu halten; wobei vorzugsweise das Halteelement ein Vorspannelement (244) aufweist, das mit dem Kolben im Eingriff ist, um den Kolben in Eingriff mit der Dissektorspitze zu drängen.

7. Chirurgische Klammervorrichtung nach Anspruch 6, wobei der Kolben einen Vorsprung (242a) aufweist, der Vorsprung in eine der beabstandeten Aussparungen aufgenommen ist, um die Dissektorspitze in einer von der ersten oder der zweiten Position zu halten; wobei vorzugsweise der distale Abschnitt des Ambosses einen Gabelkopf aufweist und der proximale Abschnitt der Dissektorspitze einen Gelenkabschnitt aufweist, der von einem Gabelkopfstift in dem Gabelkopf gehalten wird.

8. Chirurgische Klammervorrichtung nach Anspruch 7, wobei sich die proximale Fläche der Dissektorspitze an dem Gelenkabschnitt der Dissektorspitze befindet.

9. Chirurgische Klammervorrichtung nach einem vorhergehenden Anspruch, ferner aufweisend eine Griffanordnung, wobei der proximale Abschnitt des länglichen Körpers mit der Griffanordnung gekoppelt ist.

## Revendications

1. Dispositif d'agrafage chirurgical (10) comprenant :
un corps allongé (14) ayant une partie proximale et une partie distale ; et
un ensemble outil (100) définissant un axe longitudinal et comprenant :
un ensemble de cartouche (112) ;
une enclume (114, 118) comprenant une partie proximale et une partie distale, l'enclume étant couplée à l'ensemble de cartouche de telle sorte que l'ensemble outil peut passer d'une position ouverte à une position serrée ;
une pointe de dissecteur (120) comprenant un corps ayant une partie proximale (150a) et une partie distale (150b), le corps ayant une épaisseur qui diminue vers la partie distale du corps de la pointe de dissecteur, le corps étant couplé de manière mobile à l'enclume pour un mouvement entre une première position sensiblement alignée avec l'axe longitudinal et une seconde configuration définissant un angle aigu avec l'axe longitudinal ; la pointe de dissecteur comprenant une face proximale (154a) qui définit des évidements espacés (156a, 156b, 256a, 256b), et
un élément de retenue supporté sur la partie distale de l'enclume, l'élément de retenue étant mis en prise avec les évidements espacés de la pointe de dissecteur pour retenir de manière amovible la pointe de dissecteur dans l'une des première ou seconde positions.

2. Dispositif d'agrafage chirurgical selon la revendication 1, l'élément de retenue comprenant un doigt en porte-à-faux résilient (140) supporté sur la partie distale de l'enclume.

3. Dispositif d'agrafage chirurgical selon la revendication 2, le doigt en porte-à-faux supportant une protubérance (140a), la protubérance étant reçue dans l'un des évidements espacés pour retenir la pointe de dissecteur dans l'une des première ou seconde positions.

4. Dispositif d'agrafage chirurgical selon la revendication 3, la partie distale de l'enclume comprenant une chape (130) et la partie proximale de la pointe de dissecteur comprenant une partie charnière (152) qui est soutenue à l'intérieur de la chape par un axe de chape (136).

5. Dispositif d'agrafage chirurgical selon la revendication 4, la face proximale de la pointe de dissecteur étant située sur la partie charnière de la pointe de dissecteur.

6. Dispositif d'agrafage chirurgical selon la revendication 1, l'élément de retenue comprenant un piston (242) qui est mis en prise de manière libérable avec la pointe de dissecteur pour retenir la pointe de dissecteur dans l'une des première ou seconde positions ; de préférence, l'élément de retenue comprenant un élément de sollicitation (244) qui est mis en prise avec le piston pour pousser le piston en prise avec la pointe de dissecteur.

7. Dispositif d'agrafage chirurgical selon la revendication 6, le piston comprenant une protubérance (242a), la protubérance étant reçue dans l'un des évidements espacés pour retenir la pointe de dissecteur dans l'une des première ou seconde positions ; de préférence, la partie distale de l'enclume comprenant une chape et la partie proximale de la pointe de dissecteur comprenant une partie charnière qui est supportée dans la chape par un axe de chape.

8. Dispositif d'agrafage chirurgical selon la revendication 7, la face proximale de la pointe de dissecteur étant située sur la partie charnière de la pointe de dissecteur.

9. Dispositif d'agrafage chirurgical selon n'importe quelle revendication précédente, comprenant en outre un ensemble de poignée, la partie proximale du corps allongé étant couplée à l'ensemble de poignée.
